# EUROPÄISCHE PATENTANMELDUNG

(11) **EP 2 937 079 A2**
(43) Veröffentlichungstag der Anmeldung: **28.10.2015**
(21) Anmeldenummer: 14186802.6
(22) Anmeldetag: 29.09.2014
(51) Int. Cl.: A61K 9/06, A61K 31/785, A61K 8/81, A61Q 11/00, A61K 9/00, A61P 31/02, A61P 31/04

(54) **Orales Abgabesystem**

(30) Priorität: 22.04.2014 DE 202014101882 U
(71) Anmelder: Arnold, Christian, 85560 Ebersberg (DE); Armani, Armin, 82031 Grünwald (DE)
(72) Erfinder: Arnold, Christian, 85560 Ebersberg (DE); Armani, Armin, 82031 Grünwald (DE)
(74) Vertreter: advotec.

(57) **Zusammenfassung**

Die Erfindung betrifft ein orales Abgabesystem für die Behandlung und/oder Vorbeugung von erregerbedingten, pathologischen Veränderungen im Mund- und Rachenraum, umfassend Polyhexanid.

## Beschreibung

Die vorliegende Erfindung betrifft ein orales Abgabesystem für die Behandlung und/oder Vorbeugung von periodontalen, periimplantären sowie anderen bakteriellen und viralen Krankheiten oder Pilzerkrankungen im Mund- und Rachenraum sowie Halitosis (nachfolgend: erregerbedingte, pathologische Veränderungen).

Erregerbedingte, pathologische Veränderungen treten sehr häufig auf und sind der Hauptgrund für Zahnverlust/Implantatverlust und Halitosis bei Menschen in einem Alter von über 35 Jahren. Eine erregerbedingte, pathologische Veränderung kann z.B. als eine Infektion oder Entzündung der Zahnfleischtasche verstanden werden, welche, in mehreren Schritten, den Verlust des Knochens, welcher den Zahn/Implantat hält, bewirken kann. Es bestehen unterschiedliche Ausprägungen der Schwere der Krankheit. Leichtere Fälle betreffen die klinisch bezeichnete Gingivitis, während schwerwiegendere Fälle klinisch als Periodontitis/Periimplantitis bezeichnet werden.

Eine Gingivitis ist eine Entzündung der Gingiva (oder des Zahnfleisches), welche oft durch eine schlechte orale Hygiene und/oder den hormonalen Zustand des Patienten ausgelöst wird. Es wird angenommen, dass sich die unbehandelte Gingivitis in eine Periodontitis/Periimplantitis entwickelt. Die Periodontitis ist eine bakterielle Krankheit, welche das Zahnfleischgewebe, die Zähne, Implantate und den Knochen, welcher die Zähne/Implantate umgibt, angreift.

Die Mundhöhle ist eine im Wesentlichen aerobe Umgebung, welche durch den Speichel durchflossen ist. Im Gegensatz hierzu ist die periodontale/periimplantäre Mikroumgebung eher anaerob und wird durch ein Plasmafiltrat durchflossen, welches als "gingivale Sulkusflüssigkeit" bezeichnet wird. Das Wachstum von Mikroorganismen innerhalb dieser Mikroumgebung wird als verantwortlich für das Auftreten einer erregerbedingten, pathologischen Veränderung angesehen. Daher ist die Behandlung dieser Veränderung auf die Überwachung und Beeinflussung dieses Wachstums gerichtet.

Versuche zur Behandlung von erregerbedingten, pathologischen Veränderungen durch Wirkstoffe, z. B. antibakterielle Wirkstoffe, welche in die Mundhöhle gegeben werden, stellten sich in der Regel als unwirksam heraus, da die periodontale/periimplantäre Tasche im Wesentlichen nicht zugänglich ist. Andererseits führt die systemische Verabreichung von Antibiotika nur zu einem geringen Erfolg in der Behandlung von periodontalen Krankheiten.

Antibakterielle Wirkstoffe wie Chlorhexidin und quarternäre Ammoniumsalze in Form von Mundspülungen haben sich zwar als einigermaßen wirkungsvoll bei der Prophylaxe/Behandlung von erregerbedingten, pathologischen Veränderungen erwiesen. Diese Wirkstoffe haben jedoch verschiedene Nachteile. So treten häufig Nebenwirkungen, wie Verfärbung der Zähne, Zunge, Schleimhäute oder von Zahnersatz auf. Ferner weisen diese Wirkstoffe häufig üblen Geschmack auf und beeinträchtigen die Geschmacksempfindung des Patienten. Ferner kommt es bei diesen Wirkstoffen häufig zu Störungen der Wundheilung. Zudem tritt häufig auch Resorption der Wirkstoffe über die Schleimhäute oder über den Magen-Darm-Trakt auf, was zu systemischen Wirkungen führen kann. Ferner entstehen bei den genannten Wirkstoffen häufig toxische Abbauprodukte. Ein weiterer Nachteil besteht darin, dass die genannten Wirkstoffe meist in sehr hohen Konzentrationen eingesetzt werden müssen, um eine entsprechende Wirkung zu erzielen. Ferner wurde allergogenes Potenzial beobachtet. Außerdem wirken die genannten Wirkstoffe häufig irritativ und sind nicht besonders lange haltbar. Ferner wurde beobachtet, dass bei diesen Wirkstoffen die Wirkung durch Blut bzw. Eiweiß beeinflusst wird.

Es wurden pharmazeutische Zusammensetzungen, welche eine Freisetzung von Wirkstoffen aufweisen und welche in der Lage sind, in die periodontale Kavität eingesetzt zu werden und welche langsam einen antimikrobiellen Wirkstoff freisetzen, entwickelt. Zum Beispiel offenbaren US 4,764,377 und US 4,892,736 das Einbringen von Tetracyclin in nicht abbaubare polymere Fasern, welche um die Zähne herum gewickelt werden können und das Antibiotikum in der periodontalen Kavität für mehrere Tage freisetzen. Die Fasern müssen jedoch mit einem Klebstoff an ihrem Platz fixiert werden und am Ende der Behandlungsmethode wieder entfernt werden.

US 4,569,837 offenbart die Verwendung von wasserlöslichen polymeren Substanzen (z. B. Methylcellulose, Gelatine etc.) als eine polymere Matrix für ein periodontales Implantat.

US 5,002,769 offenbart ein biologisch abbaubares System zur oralen Verabreichung mit verzögerter Freisetzung für die Behandlung der periodontalen Krankheiten. Der Wirkstoff ist in eine Matrix von hydrolysierter Gelatine, welche mit Glutaraldehyd vernetzt ist, eingebettet.

Die oben beschriebenen Zusammensetzungen haben unterschiedliche Wirksamkeiten beim Reduzieren der bakteriellen Menge der periodontalen Tasche und im Reduzieren der Taschentiefe. Darüber hinaus weisen diese Zusammensetzungen häufig die o.g. Nachteile auf.

Der vorliegenden Erfindung liegt die Aufgabe zugrunde, ein wirksames Abgabesystem für die Behandlung und/oder Vorbeugung von erregerbedingten, pathologischen Veränderungen zur Verfügung zu stellen, welches einfach in der Anwendung und gleichzeitig effektiv ist.

Die Aufgabe wird gelöst durch ein orales Abgabesystem der eingangs genannten Art, welches Polyhexanid umfasst.

Es hat sich herausgestellt, dass Polyhexanid besonders effektiv beim Einsatz oraler Abgabesysteme bei der Behandlung und/oder Vorbeugung von periodontalen Krankheiten ist. Polyhexanid lässt sich insbesondere bei solchen Abgabesystemen einsetzen, bei welchen eine langsame Abgabe im Bereich der Zahnfleischtaschen erzielt werden soll, sodass eine verlängerte Einwirkzeit erreicht werden kann.

Bei einer bevorzugten Variante des oralen Abgabesystems liegt dieses in einer festen Darreichungsform vor. Eine feste Darreichungsform hat insbesondere Vorteile bei der Verwendung des Systems.

Bei einer bevorzugten Ausführungsform des oralen Abgabesystems liegt dies in Form eines Kaugummis vor. Kaugummis haben u.a. den Vorteil, dass sie dem bei Polyhexanid beobachteten verzögerten Wirkeintritt entgegenwirken, da sie vom Patienten ohne Probleme über einen längeren Zeitraum benutzt werden können. Dies ist beispielsweise bei Mundspülungen problematisch.

Kaugummis sind im Allgemeinen aus folgenden Rohstoffgruppen zusammengesetzt: Gum base oder Kaumasse, Weichmacher, Füllstoffe, Gleitmittel, Fette, Emulgatoren, Aromen, Farbstoffe, Antioxidantien und Genuss-Säuren zum Aromatisieren. Als Kaumasse können entweder Naturstoffe, wie Chicle, Gutta-percha, Latex, Benzoeharze oder Gummi arabicum, oder konsistenzgebende, synthetische Thermoplaste, wie Polyvinylacetat in Mengen von bis zu 65% der Kaumasse, Poly-Butadien-Styrol, Polyisobutylen, Isopren, Polyvinylether und Polyethylen, verwendet werden.

Typische Weichmacher sind Emulgatoren, Harze, Wachse oder Glucitol. Typische Füllstoffe sind Magnesiumstearat, Kreide, Calciumcarbonat, Silicat oder Cellulosen. Die Füllstoffe bzw. technischen Hilfsstoffe erhalten die Rieselfähigkeit bzw. verhindern das Verkleben der Partikel bei niedrigem Druck. Als Gleitmittel, Fette oder Emulgatoren kommen typischerweise Mineralöle, mikrokristalline Wachse oder pflanzliche Öle zum Einsatz. Diese Hilfsstoffe verhindern das Verkleben der Werkzeuge bzw. an den Werkzeugen.

Herkömmliche Kaugummis können aufgrund ihres hohen Zuckeranteils stark kariesfördernd sein, massieren aber auch das Zahnfleisch und die Speicheldrüsen bei Mundtrockenheit. Durch die zugesetzten Aromastoffe wirken Kaugummis auch erfrischend, belebend und/oder durststillend.

Um die oben genannte kariesfördernde Wirkung von Zucker in Kaugummis zu vermeiden, werden seit langem zuckerfreie Kaugummis auf dem Markt gebracht. Bei diesen wird der Zucker gegen Zuckeraustauschstoffe ersetzt. Dies sind vor allem Sorbit und Xylit.

Die erfindungsgemäßen Kaugummis dienen der unterstützenden Zahn- und Mundhygiene sowie der Behandlung/Vorbeugung von erregerbedingten, pathologischen Veränderungen. Sie sind insbesondere für unterwegs geeignet, wenn keine Möglichkeit zum Zähneputzen besteht. Die erfindungsgemäßen Kaugummis sind gewöhnlich zuckerfrei und enthalten, vergleichbar mit Zahnpasta, Spuren von Mineralien für die Regeneration der Zähne. Der erfindungsgemäße Kaugummi kann ebenfalls mindestens ein Schleifinittel oder mindestens einen Abrasivstoff, insbesondere Calziumcarbonat, Calziumphosphate, Metaphosphate, Kieselsäure, Aluminiumoxide, Silicate und/oder Talkum umfassen.

Des Weiteren kann der erfindungsgemäße Kaugummi mindestens einen Suspensionsstoff oder ein Feuchthaltemittel, insbesondere Wasser, Glycerin, Propylenglykol und/oder Sorbitsirup, sowie mindestens ein Verdickungs-, Stabilisierungs- und/oder Bindemittel, insbesondere Gele, Stärken, Alginate, Öle und/oder Cellulose Gum enthalten.

Zur Verbesserung der Geschmackseigenschaften können dem Kaugummi mindestens ein aromatischer Stoff, mindestens ein Süssungsmittel und/oder mindestens ein Zuckeraustauschstoff, insbesondere Menthol, Pfefferminzöl, Natriumsaccharin, Aspartame, Acesulfam, Sorbit, Malit, Xylit und/oder Fructose zugegeben werden.

Zudem kann der erfindungsgemäße Kaugummi weitere chemische Additive, Farbstoffe und/oder ph-Regulatoren, insbesondere Fluoride, Adstringentien, Entzündungshemmer, Desensiblisierungsmittel, Vitamine, Panthenol, Weißpigmente und/oder Natriumhydroxid enthalten.

Durch das erfindungsgemäße orale Abgabesystem in Form eines Kaugummis kann das Polyhexanid besonders effektiv an die Wirkorte, insbesondere in die Zahnfleischtaschen gelangen. Durch den Kauvorgang wird der Kaugummi in die Zahnfleischtasche gepresst, wo dann das Polyhexanid abgegeben wird.

Bei einer weiteren Ausführungsform des erfindungsgemäßen Abgabesystems liegt dieses in Form eines Chips oder Filmes vor, umfassend ein biologisch abbaubares oder bioerodierbares pharmazeutisch annehmbares Polymer.

Der erfindungsgemäße Chip bzw. Film ist zum Implantieren in eine periodontale Tasche geeignet und in der Lage, erregerbedingte, pathologische Veränderungen zu behandeln, bei welchen eine verzögerte Abgabe von Polyhexanid gewünscht ist. Die Tasche kann eine natürliche Tasche sein, kann durch einen Krankheitszustand bedingt sein oder kann absichtlich geöffnet sein als Teil der Behandlung. Nach ihrer Implantation wird der Chip bzw. Film weich, quillt auf und verändert sich in eine weiche Paste, welche in der Tasche haftet.

Vorzugsweise kann der Chip bzw. Film mindestens ein Vernetzungsmittel umfassen, welches in einer Menge vorhanden ist, die ausreicht, um das Polymer wasserunlöslich zu machen, während die Freisetzung vom Polyhexanid aus dem Abgabesystem erlaubt wird.

Der erfindungsgemäße Film bzw. Chip enthält vorzugsweise ein oberflächenaktives Mittel, welches vorzugsweise ausgewählt ist aus anionischen, kationischen und nichtionischen oberflächenaktiven Mitteln. Die nichtionischen oberflächenaktiven Mittel können ausgewählt sein aus Polyoxyethylen-Sorbitanfettsäureestern (Polysorbat) und Sorbitan-Fettsäureestern.

Der erfindungsgemäße Chip bzw. Film ist vorzugsweise angepasst zur Verabreichung in eine periodontale Tasche mit in-vivo Freisetzungseigenschaften, welche darauf abzielen, die Tiefe einer periodontale Tasche eines Patienten zu reduzieren.

Mit Vorteil liegt der erfindungsgemäße Chip bzw. Film in solch einer Form vor, dass er in der periodontale Tasche biologisch abgebaut wird, wobei er dadurch weich wird und an der periodontalen Tasche haftet und worin er, wenn er einmal in einer periodontalen Tasche eingesetzt wurde, graduell das Polyhexanid über einen Zeitraum von mindestens ca. 24 Stunden freisetzt, währenddessen sich der Chip bzw. Film in ein weiches Material umwandelt. Der Chip dient also als Abgabemedium von Polyhexanid als antimikrobieller Wirkstoff zur Applikation in den Sulkus oder Zahnfleischtasche. Die Größe eines erfindungsgemäßen Chips/Films liegt in der Regel bei 3x3 - 10x10 mm. In der Regel ist die Basis des Chips/Films eine mit Glutaraldehyd vernetzte Gelatine. Es hat sicher herausgestellt, dass vernetzte Gelatine besonders geeignet ist zur verzögerten Freisetzung von Polyhexanid.

Mit Vorteil ist das Polymer ausgewählt aus wasserlöslichem Protein, Zellulose oder Zellulose-Derivat, Stärke oder Stärke-Derivat, Glycerylmonostearat, Carbomer, PVP (Polyvinylpyrrolidon), Gummi, Akaziengummi, Guargummi, Polyvinylalkohol, Polyhydroxyethylmethacrylat, Polyhydroxymethylmethacrylatacrylsäure, Polyacrylamid, Polyethylenglykolen, Polyessigsäure, Polyglykolsäure, Copolymere von Polyessigsäure und Polyglykolsäure, Polyanhydride und Polyorthoestern. Das wasserlösliche Protein ist vorzugweise ausgewählt aus der Gruppe, bestehend aus Gelatine, Collagen, Albumin, einem Enzym und Fibrinogen.

Bei einer weiteren Ausführungsform des erfindungsgemäßen Systems liegt dieses als Gel oder Salbe vor. In dieser Form wird es in der Regel in die Zahnfleischtaschen verbracht, wo es Polyhexanid an die Umgebung abgibt.

Das erfindungsgemäße Gel bzw. Salbe dient zur intraoralen Anwendung, insbesondere zur Applikation in den Sulkus oder in eine Zahnfleischtasche. Das Polyhexanid wirkt hier wiederum als antimikrobieller Wirkstoff. Das erfindungsgemäße Gel/Salbe kann auf herkömmlicher Ethanol-/Glycerol-/Macrogol-Verbindung basieren.

Das erfindungsgemäße Gel bzw. die erfindungsgemäße Salbe kann sowohl als Fertigpräparat als auch als Mischsystem vorliegen. Beim Vorliegen eines Mischsystems werden bestimmte Komponenten erst kurz vor der Anwendung gemischt und an die erkrankte Stelle gebracht. Der Vorteil hierbei besteht u.a. darin, dass ein Mischsystem nach dem Mischen gut verarbeitbar ist. So kann bei einer Ausführungsform nach dem Mischen zunächst eine viskose Masse vorliegen, die erst nach dem Verbringen in den Mund aushärtet. Dies erleichtert die Anwendung erheblich. Das Mischsystem kann beispielsweise in Form einer Mischkapsel vorliegen.

Das erfindungsgemäße orale Abgabesystem hat gegenüber den bekannten antibakteriellen Wirkstoffen, wie beispielsweise Chlorhexidin gravierende Vorteile. So ist das im erfindungsgemäßen Abgabesystem eingesetzte Polyhexanid nicht zytotoxisch. Zudem sind keine Nebenwirkungen, wie Verfärbung der Zähne, Zunge, Schleimhäute oder von Zahnersatz zu beobachten. Das eingesetzte Polyhexanid ist geschmacksneutral und verursacht keine Geschmacksbeeinträchtigungen. Ferner wird die Wundheilung nicht gestört und die Fibrinbildung wird reduziert. Ferner ist keine Resorption über Schleimhäute oder über den Magen-Darm-Trakt bekannt. Ferner bilden sich bei der Anwendung keine toxischen Abbauprodukte. Ein weiterer erheblicher Vorteil besteht darin, dass die Wirkkonzentration von Polyhexanid um ein Vielfaches niedriger als beispielsweise von Chlorhexidin ist. Ferner birgt Polyhexanid kein allergogenes Potenzial, ist nicht irritativ. Polyhexanid ist - soweit bekannt - nicht sensibilisierend. Die Haltbarkeit von Polyhexanid ist ebenfalls länger als bei den herkömmlichen Wirkstoffen. Zudem wurde keine Resistenzbildung beobachtet. Polyhexanid hat ein breites Wirkspektrum und zeigt einen sehr geringen Eiweiß- und Blutfehler (Wirkung wird durch Eiweiß oder Blut kaum beeinträchtigt).

## Patentansprüche

1. Orales Abgabesystem für die Behandlung und/oder Vorbeugung von erregerbedingten, pathologischen Veränderungen im Mund- und Rachenraum, umfassend Polyhexanid.

2. Orales Abgabesystem nach Anspruch 1, **dadurch gekennzeichnet, dass** das System in einer festen Darreichungsform vorliegt.

3. Orales Abgabesystem nach einem der Ansprüche 1 oder 2, **dadurch gekennzeichnet, dass** es in Form eines Kaugummis vorliegt.

4. Orales Abgabesystem nach Anspruch 3, **gekennzeichnet durch** mindestens einen Suspensionsstoff oder ein Feuchthaltemittel, insbesondere Wasser, Glycerin, Propylenglykol und/oder Sorbitsirup.

5. Orales Abgabesystem nach einem der Ansprüche 3 oder 4, **gekennzeichnet durch** mindestens ein Verdickungs-, Stabilisierungs- und/oder Bindemittel, insbesondere Gele, Stärken, Alginate, Öle und/oder Cellulose Gum.

6. Orales Abgabesystem nach einem der Ansprüche 3 bis 5, **gekennzeichnet durch** mindestens einen aromatischen Stoff, Süssungsmittel und/oder Zuckeraustauschstoffe, insbesondere Menthol, Pfefferminzöl, Natriumsaccharin, Aspartam, Acesulfam, Sorbit, Malit, Xylit und/oder Fructose.

7. Orales Abgabesystem nach einem der Ansprüche 3 bis 6, **gekennzeichnet durch** weitere chemische Additive, Farbstoffe und/oder ph-Regulatoren, insbesondere Fluoride, Adstringentien, Desensibilisierungsmittel, Vitamine, Panthenol, Weißpigmente und/oder Natriumhydroxid.

8. Orales Abgabesystem nach einen der Ansprüche 1 oder 2, **dadurch gekennzeichnet, dass** es in Form eines Chips oder Filmes vorliegt, umfassend ein biologisch abbaubares oder bioerodierbares pharmazeutisch annehmbares Polymer.

9. Orales Abgabesystem nach einem der Ansprüche 1, 2 oder 8, **dadurch gekennzeichnet, dass** es ein Vernetzungsmittel umfasst, welches in einer Menge vorhanden ist, die ausreichend ist, um das Polymer wasserunlöslich zu machen, während die Freisetzung von Polyhexamid aus dem System erlaubt wird.

10. Orales Abgabesystem nach einem der Ansprüche 1, 2, 8 oder 9, **dadurch gekennzeichnet, dass** es ferner ein oberflächenaktives Mittel umfasst, vorzugsweise ausgewählt aus anionischen, kationischen und nichtionischen oberflächenaktiven Mitteln, wobei die nichtionischen oberflächenaktiven Mittel vorzugweise ausgewählt sind aus Polyoxyethylen-Sorbitanfettsäureestern (Polysorbat) und SorbitanFettsäureestern.

11. Orales Abgabesystem nach einem der Ansprüche 1, 2, 8, 9 oder 10, **dadurch gekennzeichnet, dass** es angepasst ist zur Verabreichung in eine periodontale Tasche mit in-vivo Freisetzungseigenschaften, welche darauf abziehen, die Tiefe einer periodontalen Tasche eines Patienten zu reduzieren.

12. Orales Abgabesystem nach einem der Ansprüche 1, 2, 8, 9, 10 oder 11, **dadurch gekennzeichnet, dass** es in einer Form vorliegt, dass es in der periodontalen Tasche biologisch abgebaut wird, wobei es dadurch weich wird und an der periodontalen Tasche haftet, und worin es, graduell Polyhexamid über einen Zeitraum von mindestens ca. 24 Stunden freisetzt.

13. Orales Abgabesystem nach einen der Ansprüche 1, 2, 8, 9, 10, 11 oder 12, **dadurch gekennzeichnet, dass** das Polymer ausgewählt ist aus wasserlöslichem Protein, Zellulose oder Zellulose-Derivat, Stärke oder Stärke-Derivat, Glycerylmonostearat, Carbomer, PVP (Polyvinylpyrrolidon), Gummi, Akaziengummi, Guargummi, Polyvinylal-kohol, Polyhydroxyethylmethacrylat, Polyhydroxymethylmethacrylatacrylsäure, Polyacrylamid, Polyethylenglykolen, Polyessigsäure, Polyglykolsäure, Copolymere von Polyessigsäure und Polyglykolsäure, Polyanhydride und Polyorthoestern.

14. Orales Abgabesystem nach Anspruch 13, **dadurch gekennzeichnet, dass** das wasserlösliche Protein ausgewählt ist aus der Gruppe, bestehend aus Gelatine, Collagen, Albumin, einem Enzym, Macrogolol, Zellulose, Ethanolen und Fibrinogen.

15. Orales Abgabesystem nach einem der Ansprüche 1, 2, 8, 9, 10, 11, 12, 13 oder 14, **dadurch gekennzeichnet, dass** das Polymer durch einen Vernetzungsprozess in Gegenwart eines Vernetzungsmittels, vorzugsweise Glutaraldehyd, vernetzt wird.

16. Orales Abgabesystem nach Anspruch 1, **dadurch gekennzeichnet, dass** das System als Gel oder Salbe vorliegt.

17. Orales Abgabesystem nach Anspruch 16, **dadurch gekennzeichnet, dass** es als Mischsystem zur Zubereitung des Gels oder der Salbe vorliegt.
